# EUROPEAN PATENT SPECIFICATION

(11) **EP 3 191 145 B1**
(45) Date of publication and mention of the grant of the patent: **23.12.2020**
(21) Application number: 15767389.8
(22) Date of filing: 08.09.2015
(51) Int. Cl.: A61L 26/00

(54) **MICRONIZED PLACENTAL TISSUE COMPOSITIONS**
MIKRONISIERTE PLAZENTAGEWEBEZUSAMMENSETZUNGEN
COMPOSITIONS DE TISSUS PLACENTAIRES MICRONISÉS

(30) Priority: 09.09.2014 US 201462048218 P
(43) Date of publication of application: 19.07.2017
(73) Proprietor: MiMedx Group, Inc., Marietta, GA 30062 (US)
(72) Inventor: MCQUEEN, Adam, Marietta, GA 30062 (US); BURROWS, Frank, Marietta, GA 30062 (US); KOOB, Thomas, Marietta, GA 30062 (US)
(74) Representative: Gibson, Mark
(86) International application number: PCT/US2015/049033
(87) International publication number: WO 2016/040385

(56) References cited:
- WO-A1-2014/028327
- WO-A1-2014/144525
- WO-A2-2012/003377
- WO-A2-2012/112410
- US-A1- 2007 071 828

## Description

### FIELD OF ART

The present invention relates generally to the field of wound care.

### BACKGROUND

Skin is a protective barrier that shields the body from many bacteria, viruses, fungi, and other microbes present in the environment. A wound results in a break in the continuity of a subject's skin. Wounds can be caused, for example, by surgery, gun shots, puncture wounds, falls or other accidents, sports-related injuries, burns, animal bites or scratches, or other trauma. Any break in the skin, whether caused by surgery or a scraped knee, creates an unobstructed route for pathogens to enter the bloodstream. As a result, any wound, if not properly cleaned and dressed, can become infected. The bacteria *Staphylococcus aureus* and group A *Streptococcus* are some of the most common causes of wound infection. Wound infections can cause pain, swelling, and redness. If not properly treated, infections may spread, resulting in more serious and/or life threatening conditions, such as sepsis. Wound infections following surgery remain one of the most prevalent hospital-acquired infections and are a significant cause of post-operative morbidity and mortality.

WO 2012/112410 describes compositions comprising micronized placental components, namely amnion, chorion, intermediate tissue layer or any combination thereof, wherein the composition is not composed solely of micronized amnion.

### SUMMARY

There is a need inside and outside the hospital setting for improved wound healing compositions, procedures, and apparatuses. There is a need for improved products that protect wounds as they heal. For example, there is a need for a product that is easy to apply, is biocompatible, promotes the body's natural healing process, seals the wound to protect against microbes, and/or provides long-term (e.g., multi-day) protection. The scope of the invention is defined by the claims. Any references in the description to methods of treatment refer to the composition for use in a method for treatment of the human body by therapy.

Described herein is a unique approach to using placental tissue components in combination with an agent that maintains the placental tissue components at the site of application. In one aspect, the invention relates to a suspension of micronized placental tissue in a flowable composition that, when applied to tissue, forms a coherent mass that retains its position at the site of application. The coherent mass is formed by gelation of a phase-transfer agent (hydrogel, poloxamer). Such compositions are useful in wound healing and other medical applications.

Described herein are compositions composed of micronized placental components and a biocompatible agent that is flowable prior to application and becomes a gel, semi-solid, or solid after application to a wound or tissue, as well as pharmaceutical compositions or kits thereof. The compositions have numerous medical applications, including treatment of wounds. The wound can be a chronic wound or an acute wound. Systems and methods for making and using the compositions and kits are also described herein.

Placental tissue is typically harvested after an elective Cesarean surgery. The placenta is composed of the umbilical cord and amniotic sac. The amniotic sac, commonly referred to as the amniotic membrane, contains the amniotic fluid and protects the fetal environment. The amniotic sac has two primary layers of tissue, amnion and chorion. Amnion tissue is the innermost layer of the amniotic sac and is in direct contact with the amniotic fluid. Histological evaluation indicates that the membrane layers of the amnion consist of a single layer of epithelium cells, thin reticular fibers (basement membrane), a thick compact layer, and a fibroblast layer. The fibrous layer of amnion (*i.e*., the basement membrane) contains collagen types IV, V, and VII, and cell-adhesion bio-active factors including fibronectin and laminins. Such components may assist in wound healing if applied in a controlled manner at a wound site.

According to one aspect of the invention, there is provided a composition formulated for injection comprising micronized amnion, optionally further comprising micronized placental tissue selected from the group consisting of chorion, intermediate tissue layer, or any combination thereof and a biocompatible gelation agent in a suitable excipient, wherein the biocompatible gelation agent comprises:
a thermosensitive sol-gel reversible hydrogel, preferably
a poloxamer, wherein the composition is in a sol (liquid) phase at about 0 °C to about 20 °C and transitions to a gel (solid) phase at about 25 °C to about 40 °C.

According to a further aspect of the invention, there is provided a system for injecting micronized amnion, the system comprising:
a first composition comprising micronized amnion, optionally further comprising placental tissue selected from the group consisting of micronized chorion, intermediate tissue layer, or any combination thereof, wherein the first composition is in a flowable form;
a second composition comprising a biocompatible gelation agent in a suitable excipient, wherein the second composition is in a flowable form; and
an administration device, comprising a first chamber having the first composition disposed therein, a second chamber having the second composition stored therein, and one or more apertures configured to expel the first and second compositions therefrom;
wherein the biocompatible gelation agent comprises:
   a thermosensitive sol-gel reversible hydrogel, preferably
   a poloxamer, wherein the composition is in a sol (liquid) phase at about 0 °C to about 20 °C and transitions to a gel (solid) phase at about 25 °C to about 40 °C.

Disclosed herein are compositions useful in treating a wound. One aspect disclosed herein relates to a composition comprising micronized amnion, a biocompatible gelation agent, and optionally an extract from micronized placental components in a suitable excipient. In one aspect, provided is a system comprising the composition and a device for delivering or applying the composition.

Disclosed herein is a composition comprising micronized amnion, optionally further comprising micronized placental tissue selected from the group consisting of chorion, intermediate tissue layer, or any combination thereof and a biocompatible gelation agent in a suitable excipient, wherein the composition is in a flowable form prior to application to a subject. In one embodiment, the composition is a liquid, a gel, or a paste prior to application. In one embodiment, the composition becomes a solid, a semi-solid, or a gel after application to a subject.

Also disclosed herein is a system for dressing a wound, the system comprising a first composition comprising micronized amnion, optionally further comprising micronized placental tissue selected from the group consisting of chorion, intermediate tissue layer, or any combination thereof, wherein the first composition is in a flowable form; a second composition comprising a biocompatible gelation agent in a suitable excipient, wherein the second composition is in a flowable form; and an administration device, comprising a first chamber having the first composition disposed therein, a second chamber having the second composition stored therein, and one or more apertures configured to expel the first and second compositions therefrom. In one embodiment of the disclosure, the administration device comprises a dual-chamber sprayer. In one embodiment, the administration device comprises a double syringe.

The biocompatible gelation agent comprises a thermosensitive sol-gel reversible hydrogel. In one embodiment, the biocompatible gelation agent is a poloxamer.

In one embodiment, the micronized placental tissue and the biocompatible gelation agent are mixed immediately prior to injection.

In one embodiment, the micronized amnion and optional micronized placental tissue have a particle size of less than 100 µm.

The method for dressing a wound to promote healing may comprise providing a system for dressing a wound comprising a first composition comprising micronized amnion, optionally further comprising micronized placental tissue selected from the group consisting of chorion, intermediate tissue layer, or any combination thereof, wherein the first composition is in a flowable form; a second composition comprising a biocompatible adhesive or gelation agent in a suitable excipient, wherein the second composition is in a flowable form; said compositions being present in an administration device; positioning the administration device over a subject at the site of a wound; and applying a force to the administration device to simultaneously apply the first and second compositions onto the wound, wherein applying a force to the administration device causes a pressure and/or a volume to change within both the first and the second chambers, resulting in expulsion of the first composition from the first chamber and expulsion of the second composition from the second chamber. The first and second compositions mix within a chamber or tube prior to expulsion from a proximal aperture of the administration device. The first composition is expelled from a first nozzle or aperture and the second composition is expelled from a second nozzle or aperture, and wherein mixing of the first and second compositions occurs only external to the administration device.

Also disclosed is a kit comprising (a) a first composition comprising micronized amnion, optionally further comprising micronized placental tissue selected from the group of chorion, intermediate tissue layer, and any combination thereof in a suitable excipient, and (b) a second composition comprising a biocompatible gelation agent in a suitable excipient. In one embodiment of the disclosure, the micronized placental tissue and the biocompatible gelation agent are separated until immediately prior to administration. In one aspect of the disclosure, the kit further comprises one or more devices for delivering or applying one or both compositions.

In some aspects, the compositions described herein further comprise an additional biologically active agent, such as cytokines, chemokines, other naturally occurring regulatory proteins and enzymes, extracellular matrix components, and/or other agents described herein.

Also disclosed are methods and devices of making or using the compositions or kits.

Several of the advantages of this invention will be set forth in part in the description which follows, and in part will be obvious from the description, or may be learned by practice of the aspects described below. The advantages described below will be realized and attained by means of the elements and combinations particularly pointed out in the appended claims. It is to be understood that both the foregoing general description and the following detailed description are exemplary and explanatory only and are not restrictive.

### BRIEF DESCRIPTION OF THE DRAWINGS

Certain features, aspects, and advantages of the present technology are further described herein with reference to the accompanying drawings, which form part of the present disclosure.. Unless specifically indicated, the features of the drawings are not drawn to scale.

FIGs. 7 and 12-13 each schematically illustrate an embodiment of an administration apparatus used to administer one or both of a micronized placental tissue composition and a sealant.

FIG. 14 is an overview flow chart of one embodiment of the process for making the micronized compositions described herein.

### DETAILED DESCRIPTION

The detailed description is divided into various sections only for the reader's convenience, and disclosure found in any section may be combined with that in another section. It is also to be understood that the terminology used herein is for the purpose of describing particular embodiments only, and is not intended to be limiting.

### Definitions

Unless defined otherwise, all technical and scientific terms used herein have the same meaning as commonly understood by one of ordinary skill in the art to which this invention belongs. As used herein the following terms have the following meanings.

As used herein, the term "exemplary" means "serving as an example or illustration" and should not necessarily be construed as preferred or advantageous over other embodiments. Aspects of the disclosure, as described and illustrated herein, can be arranged, combined, and designed in a variety of different configurations, all of which are explicitly contemplated and form part of this disclosure.

As used herein and in the appended claims, the singular forms "a", "an", and "the" include plural referents unless the context clearly dictates otherwise. Thus, for example, reference to "a bioactive agent" includes mixtures of two or more such agents. At times, the claims and/or disclosure may include terms such as "a plurality," "one or more," or "at least one;" however, the absence of such terms is not intended to mean, and should not be interpreted to mean, that a plurality is not conceived.

A "plurality" as used herein refers to more than one. For example, a plurality of excipients may be two, three, four, five, or more excipients.

The term "about" when used before a numerical designation, *e.g*., time, amount, etc., including when used before a numerical range, indicates approximations which may vary by (+) or (-) 10%, 5%, or 1%.

"Comprising" or "comprises" is intended to mean that the systems, compositions, and methods include the recited elements, and may additionally include any other elements. "Consisting essentially of' shall mean that the systems, compositions, and methods include the recited elements and exclude other elements of any essential significance to the combination for the stated purpose. Thus, a system, compositions, or method consisting essentially of the elements as defined herein would not exclude other materials or steps that do not materially affect the basic and novel characteristic(s) of the claimed invention. "Consisting of' shall mean that the systems, compositions, and methods include the recited elements and exclude anything more than trivial or inconsequential elements or steps. Embodiments defined by each of these transitional terms are within the scope of this disclosure.

"Optional" or "optionally" may mean that the subsequently described event or circumstance can or cannot occur, and that the description includes instances where the event or circumstance occurs and instances where it does not. For example, the phrase "optionally cleaning step" means that the cleaning step may or may not be performed. Similarly, the terms may mean that a subsequently described component or feature is present in some embodiments and not present in others. Absence of the term should not be interpreted to mean that a particularly recited event, component, or feature is necessary unless specifically identified as such.

The term "subject" as used herein is any vertebrate organism including but not limited to mammalian subjects such as humans, farm animals, domesticated pets and the like.

The term "amnion" as used herein includes amniotic membrane where the intermediate tissue layer is intact or has been substantially removed.

The term "placental tissue" refers to any and all of the well-known components of the placenta including but not limited to amnion, chorion, Wharton's Jelly, and the like. In one preferred embodiment, the placental tissue does not include any of the umbilical cord components (e.g., Wharton's jelly, umbilical cord vein and artery, and surrounding membrane).

The term "treatment" or "treating", to the extent it relates to a disease or condition, includes preventing the disease or condition from occurring, inhibiting the disease or condition, eliminating the disease or condition, and/or relieving one or more symptoms of the disease or condition.

The term "biocompatible" as used herein refers to a material that is suitable for administration to a subject, such as application topically to or implantation or injection into the subject. In various aspects, a biocompatible material does not cause toxic or injurious effects once administered to the subject.

The term "biocompatible gelation agent" refers to any agent that is liquid prior to application to a wound or tissue (e.g., at room temperature or colder, or prior to exposure to air) and becomes a gel, semi-solid, or solid after application (e.g., at body temperature or after exposure to air). Nonlimiting examples are provided below.

The term "flowable" as used herein refers to a form of the composition having a viscosity such that it is able to flow, e.g. a liquid, gel, paste, thixotropic agent, and the like. Preferably, the composition is flowable such that it can be applied to a site, e.g. via syringe, sprayer, dropper, applicator, squeeze bottle, etc.

### Abbreviations

The following abbreviations are used throughout the specification, and have the following meanings:
°C = degrees Celsius
cm = centimeter
Da = dalton
DI = de-ionized
DMSO = dimethyl sulfoxide
EDTA = ethylenediaminetetraacetic acid
M = molar concentration (mol/L)
mg = milligram
ml = milliliter
mm = millimeter
PBS = phosphate buffered saline
rpm = rotations per minute
µm = micrometer

Titles or subtitles may be used in the specification for the convenience of a reader, which are not intended to influence the scope of the present invention. Additionally, some terms used in this specification are more specifically defined below.

### I. Compositions

Described herein in one aspect are compositions comprising micronized placental components and kits thereof useful in treating a wound. One aspect relates to a composition formulated for injection comprising micronized placental components, a biocompatible gelation agent, and optionally an extract from micronized placental components in a suitable excipient. Another aspect relates to a system comprising (a) a first composition comprising micronized placental components, and optionally an extract from micronized placental components in a suitable excipient, and (b) a second composition comprising a biocompatible adhesive or gelation agent in a suitable excipient or a topical composition comprising a biocompatible adhesive or gelation agent in a suitable excipient. In one embodiment of the disclosure, the composition(s) is sprayable.

Micronized placental components and methods of preparing the micronized placental components are described, e.g., in PCT Publication No. WO 2012/112410, filed Feb. 13, 2012, U.S. Patent Application Pub. No. 2014/0050788, filed August 9, 2013, as well as U.S. Patent Application Serial Nos. 14/718,703, filed May 21, 20154, and 14/793,673, filed July 7, 2015.

Extracts from micronized placental components are described in, e.g., U.S. Patent Application Pub. No. 2014/0050788. In some aspects, the extracts comprise the growth factors and/or cytokines of the amnion, chorion, intermediate tissue layer, umbilical cord component(s), or any combination thereof. In one aspect, the extract is a saline extract, a sterile water extract, or an extract using any suitable buffer known in the art, of the micronized placental components.

In a related aspect, separation of the extract is conducted such that the remaining micronized placental components still contain a significant amount of growth factors.

The composition comprises micronized amnion. In another aspect, the composition comprises micronized amnion and micronized intermediate tissue layer. In another aspect, the composition comprises micronized amnion and micronized chorion.

Biocompatible gelation agents include thermosensitive sol-gel reversible hydrogels such as aqueous solutions of poloxamers. In one aspect, the poloxamer is a nonionic triblock copolymer composed of a central hydrophobic chain of polyoxypropylene (*e.g*., (poly(propylene oxide)) flanked by two hydrophilic chains of polyoxyethylene (*e.g*., poly(ethylene oxide)). In one aspect, poloxamer has the formula

HO(C₂H₄O)_{b}(C₃H₆O)ₐ(C₂H₄O)_{b}OH

wherein a is from 10 to 100, 20 to 80, 25 to 70, or 25 to 70, or from 50 to 70; b is from 5 to 250, 10 to 225, 20 to 200, 50 to 200, 100 to 200, or 150 to 200. In another aspect, the poloxamer has a molecular weight from 2,000 to 15,000, 3,000 to 14,000, or 4,000 to 12,000. Poloxamers useful herein are sold under the tradename Pluronic® manufactured by BASF. Non-limiting examples of poloxamers useful herein include, but are not limited to, Pluronic® F68, P103, P105, P123, F127, and L121.

In some aspects, the suitable excipient is a pharmaceutically acceptable aqueous excipient such as water or an aqueous carrier. Examples of pharmaceutically acceptable aqueous excipient include sterile water, saline, phosphate buffered saline, aqueous hyaluronic acid, Ringer's solution, dextrose solution, Hank's solution, and other aqueous physiologically balanced salt solutions.

Nonaqueous excipients include, fixed oils, vegetable oils such as olive oil and sesame oil, triglycerides, propylene glycol, polyethylene glycol, and injectable organic esters such as ethyl oleate can also be used.

Excipients can also contain minor amounts of additives, such as substances that enhance isotonicity or chemical stability. Examples of buffers include phosphate buffer, bicarbonate buffer and Tris buffer, while examples of preservatives include thimerosol, cresols, formalin and benzyl alcohol. In certain aspects, the pH can be modified depending upon the mode of administration. In some aspect, the composition has a pH in the physiological pH range, such as pH 7 to 9.

The amount of micronized composition in a specified case will vary according to the specific compound being utilized, the particular compositions formulated, the mode of application, and the particular situs and subject being treated. In one aspect, the micronized composition is from 0.5% to 20%, 1% to 10%, 2% to 5%, or about 3% by weight of the composition, or any range between two of the numbers (end point inclusive).

In one aspect, the extract from micronized composition is from 0.5% to 20%, 1% to 10%, 2% to 5%, or about 3% by weight of the composition, or any range between two of the numbers (end point inclusive).

In one aspect, depending on the type of the biocompatible gelation agent used, the composition described here in can comprise about 0.1-100%, 0.1-50%, or 0.1-30%, such as 0.1%, 0.25 %, 0.5%, 0.75%, 1%, 2%, 5%, 7 %, 10%, 15%, 20%, 25%, 30% of the biocompatible gelation agent based on the total weigh of the composition, or any range between two of the numbers (end point inclusive).

### Compositions comprising micronized placental components and a biocompatible gelation agent

One aspect relates to a composition comprising micronized amnion, a biocompatible gelation agent, and optionally micronized placental tissue selected from chorion, intermediate tissue layer, or any combination thereof in a suitable excipient.

The biocompatible gelation agent may be an agent that is flowable prior to application to a subject (e.g., at room temperature or colder) and becomes a gel after application to the subject (e.g., at body temperature). The biocompatible gelation agent is a hydrogel.

In another aspect, disclosed herein is a composition comprising micronized placental components and a poloxamer wherein the composition is in a sol (liquid) phase at about 0 °C to about 20 °C and transitions a gel (solid) phase at or near the body temperature or higher, such as about 25 °C to about 40 °C, or about 30 °C to about 37 °C.

In one aspect, the composition can be applied to, e.g., a wound resulting from an injury or a surgical procedure. The micronized placental components provide treatment to wound, such as facilitating wound healing, reducing or preventing inflammation of the wound. The gelation agent transitions to a gel phase to retain or otherwise localize the micronized amnion, and optionally the further micronized placental tissue, at the wound site for an extended period of time for sustained treatment of the wound. In one aspect, the wound is a result of an injury. In one aspect, the wound is a result of a surgical procedure. In one aspect, the wound has an opening on the skin and the composition can be applied to the inside and/or the opening of the wound.

### Compositions and kits comprising a composition comprising micronized placental components and a composition comprising a biocompatible gelation agent

Disclosed herein is a kit comprising (a) a first composition comprising micronized amnion, optionally comprising the further placental tissue in a suitable excipient, and (b) a second composition comprising a biocompatible gelation agent in a suitable excipient.

The first composition can be applied to, e.g., a wound to treat the wound, such as facilitating wound healing, reducing or preventing inflammation and/or infection of the wound. The second composition can be then applied over the first composition to fix or otherwise localize the micronized placental components, and optionally an extract from micronized placental components, in the first composition at the application site for an extended period of time for sustained treatment of wound. In one aspect of the disclosure, the wound is a result of an injury. In one aspect of the disclosure, the wound is a result of a surgical procedure. In one aspect of the disclosure, the wound has an opening on the skin and the first composition can be applied to the inside and/or the opening of the wound while the second composition is applied to the opening of the wound to cover the first composition.

### II. Methods for Preparation

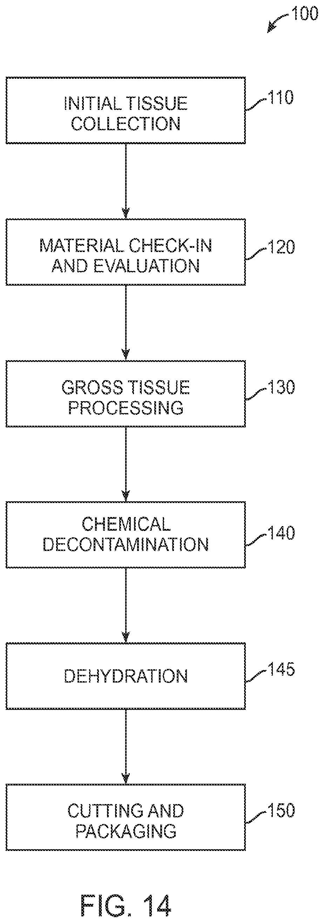

FIG. 14 depicts an overview (100) and certain aspects of the steps to harvest, process, and prepare placental material for use in the preparation of the micronized compositions described herein. More detailed descriptions and discussion regarding each individual step will follow. Initially, the placental tissue is collected from a consenting patient following an elective Cesarean surgery (step 110). The material is preserved and transported in conventional tissue preservation manner to a suitable processing location or facility for check-in and evaluation (step 120). Gross processing, handling, and separation of the tissue layers then takes place (step 130). Acceptable tissue is then decontaminated (step 140) and dehydrated (step 145). After decontamination and dehydration, the placental components (e.g., amnion, intermediate tissue layer and/or chorion individually or as grafts) are then micronized (step 150). Each step is described in detail below.

### Initial Tissue Collection (Step 110)

The components used to produce the micronized compositions described herein are derived from the placenta. The source of the placenta can vary. In one aspect, the placenta is derived from a mammal such as human or other animal including, but not limited to, cow, pig, and the like. In the case of humans, the recovery of the placenta originates in a hospital, where it is preferably collected during a Cesarean section birth. The donor, referring to the mother who is about to give birth, voluntarily submits to a comprehensive screening process designed to provide the safest tissue possible for transplantation. The screening process preferably tests for antibodies to the human immunodeficiency virus type 1 and type 2 (anti-HIV-1 and anti-HIV-2), antibodies to the hepatitis B virus (anti-HBV) hepatitis B surface antigens (HBsAg), antibodies to the hepatitis C virus (anti-HCV), antibodies to the human T-lymphotropic virus type I and type II (anti-HTLV-I, anti-HTLV-II), cytomegalovirus (CMV), and syphilis, and nucleic acid testing for human immune-deficiency virus type 1 (HIV-1) and for the hepatitis C virus (HCV), using conventional serological tests. The above list of tests is exemplary only, as more, fewer, or different tests may be desired or necessary over time or based upon the intended use of the grafts, as will be appreciated by those skilled in the art.

Based upon a review of the donor's information and screening test results, the donor will either be deemed acceptable or not. In addition, at the time of delivery, cultures are taken to determine the presence of bacteria, for example, *Clostridium* or *Streptococcus.* If the donor's information, screening tests, and the delivery cultures are all satisfactory *(i.e.,* do not indicate any risks or indicate acceptable level of risk), the donor is approved by a medical director and the tissue specimen is designated as initially eligible for further processing and evaluation.

Human placentas that meet the above selection criteria are preferably bagged in a saline solution in a sterile shipment bag and stored in a container of wet ice for shipment to a processing location or laboratory for further processing.

If the placenta is collected prior to the completion of obtaining the results from the screening tests and delivery cultures, such tissue is labeled and kept in quarantine. The placenta is approved for further processing only after the required screening assessments and delivery cultures, which declare the tissue safe for handling and use, are satisfied and final approval is obtained from a medical director.

### Material Check-in and Evaluation (Step 120)

Upon arrival at the processing center or laboratory, the shipment is opened and verified that the sterile shipment bag/container is still sealed and in the coolant, that the appropriate donor paperwork is present, and that the donor number on the paperwork matches the number on the sterile shipment bag containing the tissue. The sterile shipment bag containing the tissue is then stored in a refrigerator until ready for further processing.

### Gross Tissue Processing (Step 130)

When the tissue is ready to be processed further, the sterile supplies necessary for processing the placental tissue further are assembled in a staging area in a controlled environment and are prepared for introduction into a controlled environment. In one aspect, the placenta is processed at room temperature. If the controlled environment is a manufacturing hood, the sterile supplies are opened and placed into the hood using conventional sterilization techniques. If the controlled environment is a clean room, the sterile supplies are opened and placed on a cart covered by a sterile drape. All the work surfaces are covered by a piece of sterile drape using conventional sterilization techniques, and the sterile supplies and the processing equipment are placed onto the sterile drape, again using conventional sterilization techniques.

Processing equipment is decontaminated according to conventional and industry-approved decontamination procedures and then introduced into the controlled environment. The equipment is strategically placed within the controlled environment to minimize the chance for the equipment to come in proximity to or be inadvertently contaminated by the tissue specimen.

Next, the placenta is removed from the sterile shipment bag and transferred aseptically to a sterile processing basin within the controlled environment. The sterile basin contains hypertonic saline solution (*e.g*., 18% NaCl) that is at room or near room temperature. The placenta is gently massaged to help separate blood clots and to allow the placental tissue to reach room temperature, which facilitates the separation of the placental components from each other (*e.g*., amnion membrane and chorion). After having warmed up to ambient temperature (*e.g*., after about 10-30 minutes), the placenta is then removed from the sterile processing basin and laid flat on a processing tray with the amnion membrane layer facing down for inspection.

The placenta is examined for discoloration, debris or other contamination, odor, and signs of damage. The size of the tissue is also noted. A determination is made, at this point, as to whether the tissue is acceptable for further processing.

The amnion and chorion are next carefully separated. In one aspect, the materials and equipment used in this procedure include a processing tray, 18% saline solution, sterile 4×4 sponges, and two sterile Nalgene jars. The placenta tissue is then closely examined to find an area (typically a corner) in which the amnion can be separated from the chorion. The amnion appears as a thin, opaque layer on the chorion.

The fibroblast layer is identified by gently contacting each side of the amnion with a piece of sterile gauze or a cotton tipped applicator. The fibroblast layer will stick to the test material. The amnion is placed into the processing tray with the basement membrane layer down. Using a blunt instrument, a cell scraper, or sterile gauze, any residual blood is also removed. This step must be done with adequate care, again, so as not to tear the amnion. The cleaning of the amnion is complete once the amnion is smooth and opaque-white in appearance.

In certain aspects, the intermediate tissue layer, also referred to as the spongy layer, is substantially removed from the amnion in order to expose the fibroblast layer. The term "substantially removed" with respect to the amount of intermediate tissue layer removed is defined herein as removing greater than 90%, greater than 95%, or greater than 99% of the intermediate tissue layer from the amnion. This can be performed by peeling the intermediate tissue layer from the amnion. Alternatively, the intermediate tissue layer can be removed from the amnion by wiping the intermediate tissue layer with gauze or other suitable wipe. The resulting amnion can be subsequently decontaminated using the process described below.

In certain aspects, the epithelium layer present on the amnion is substantially removed in order to expose the basement layer of the amnion. The term "substantially removed" with respect to the amount of epithelium removed is defined herein as removing greater than 90%, greater than 95%, or greater than 99% of the epithelial cells from the amnion. The presence or absence of epithelial cells remaining on the amnion layer can be evaluated using techniques known in the art. For example, after removal of the epithelial cell layer, a representative tissue sample from the processing lot is placed onto a standard microscope examination slide. The tissue sample is then stained using Eosin Y Stain and evaluated as described below. The sample is then covered and allowed to stand. Once an adequate amount of time has passed to allow for staining, visual observation is done under magnification.

The epithelium layer can be removed by techniques known in the art. For example, the epithelium layer can be scraped off of the amnion using a cell scraper. Other techniques include, but are not limited to, freezing the membrane, physical removal using a cell scraper, or exposing the epithelial cells to nonionic detergents, anionic detergents, or nucleases. The de-epithelialized tissue is then evaluated to determine that the basement membrane has not been compromised and remains intact. This step is performed after completion of the processing step and before the tissue has been dehydrated as described in the next section. For example, a representative sample graft is removed for microscopic analysis. The tissue sample is placed onto a standard slide, stained with Eosin Y, and viewed under the microscope. If epithelium is present, it will appear as cobblestone-shaped cells.

The methods described herein do not remove all cellular components in the amnion. This technique is referred to in the art as "decellularization." Decellularization generally involves the physical and/or chemical removal of all cells present in the amnion, which includes epithelial cells and fibroblast cells. For example, although the removal of epithelial cells is optional, the fibroblast layer present in the amnion stromal layer is intact, even if the intermediate tissue layer is removed. Here, fibroblast cells are present in the fibroblast layer (fibroblast cellular layer).

### Chemical Decontamination (Step 140)

The placental tissue can be chemically decontaminated using the techniques described below. In one aspect, the amnion is decontaminated at room temperature. In one aspect, the amnion produced in step 130 (*e.g*., with or without the intermediate tissue layer) can be placed into an empty container, such as a sterile Nalgene jar or other sealable container for the next step. In one aspect, the following procedure can be used to clean the amnion. A Nalgene jar is aseptically filled with 18% saline hypertonic solution and sealed (or sealed with a top). The jar is then placed on a rocker platform and agitated for between 30 and 90 minutes, which further cleans the amnion of contaminants. If the rocker platform was not in the critical environment (*e.g*., the manufacturing hood), the Nalgene jar is returned to the controlled /sterile environment and opened. Using sterile forceps or by aseptically decanting the contents, the amnion is gently removed from the Nalgene jar containing the 18% hypertonic saline solution and placed into an empty Nalgene jar. This empty Nalgene jar with the amnion is then aseptically filled with a pre-mixed antibiotic solution. In one aspect, the premixed antibiotic solution is composed of a cocktail of antibiotics, such as Streptomycin Sulfate and Gentamicin Sulfate. Other antibiotics, such as Polymixin B Sulfate and Bacitracin, or similar antibiotics now available or available in the future, are also suitable. Additionally, it is preferred that the antibiotic solution be at room temperature when added so that it does not change the temperature of or otherwise damage the amnion. This jar or container containing the amnion and antibiotics is then sealed or closed and placed on a rocker platform and agitated for, preferably, between 60 and 90 minutes. Such rocking or agitation of the amnion within the antibiotic solution further cleans the tissue of contaminants and bacteria. Optionally, the amnion can be washed with a detergent. In one aspect, the amnion can be washed with 0.1 to 10%, 0.1 to 5%, 0.1 to 1%, or 0.5% Triton-X wash solution.

If the rocker platform was not in the critical environment (*e.g*., the manufacturing hood), the jar or container containing the amnion and antibiotics is then returned to the critical/sterile environment and opened. Using sterile forceps, the amnion is gently removed from the jar or container and placed in a sterile basin containing sterile water or normal saline (0.9% saline solution). The amnion is allowed to soak in place in the sterile water/normal saline solution for at least 10 to 15 minutes. The amnion may be slightly agitated to facilitate removal of the antibiotic solution and any other contaminants from the tissue. After at least 10 to 15 minutes, the amnion is ready to be dehydrated and processed further.

In the case of chorion, the following exemplary procedure can be used. After separation of the chorion from the amnion and removal of clotted blood from the fibrous layer, the chorion is rinsed in 18% saline solution for 15 minutes to 60 minutes. During the first rinse cycle, 18% saline is heated in a sterile container using a laboratory heating plate such that the solution temperature is approximately 48 °C. The solution is decanted, the chorion tissue is placed into the sterile container, and decanted saline solution is poured into the container. The container is sealed and placed on a rocker plate and agitated for 15 minutes to 60 minutes. After the agitation bath, the chorion tissue is removed and placed into a second heated agitation bath for an additional 15 minute- to 60 minute- rinse cycle. Optionally, the chorion tissue can be washed with a detergent (*e.g*., Triton-X wash solution) as discussed above for the decontamination of amnion. The container is sealed and agitated without heat for 15 minutes to 120 minutes. The chorion tissue is next washed with deionized water (e.g., 250 ml of DI water x 4) with vigorous motion for each rinse. The tissue is removed and placed into a container of 1x PBS with EDTA solution. The container is sealed and agitated at controlled temperature for 1-8 hours. The chorion tissue is removed and rinsed using sterile water. A visual inspection is performed to remove any remaining discolored fibrous blood material from the chorion tissue. The chorion tissue should have a cream white visual appearance with no evidence of brownish discoloration.

### Dehydration (Step 145)

In one aspect, the placental tissue or components thereof as described above, or any combination thereof can be processed into tissue grafts (*i.e*., laminates) that are subsequently micronized. In another aspect, the placental tissue or individual components thereof can be dehydrated independently and subsequently micronized alone or as a mixture of components. In one aspect, the tissue (*i.e*., individual membrane or graft) is dehydrated by chemical dehydration followed by freeze-drying. In one aspect, the chemical dehydration step is performed by contacting the amnion, chorion, and/or intermediate layer with a polar organic solvent for a sufficient time and amount in order to substantially (*i.e*., greater than 90%, greater than 95%, or greater than 99%) or completely remove residual water present in the tissue (*i.e*., dehydrate the tissue). The solvent can be protic or aprotic. Examples of polar organic solvents useful herein include, but are not limited to, alcohols, ketones, ethers, aldehydes, or any combination thereof. Specific, non-limiting examples include DMSO, acetone, tetrahydrofuran, ethanol, isopropanol, or any combination thereof. In one aspect, the placental tissue is contacted with a polar organic solvent at room temperature. No additional steps are required, and the tissue can be freeze-dried directly as discussed below.

After chemical dehydration, the tissue is freeze-dried in order to remove any residual water and polar organic solvent. In one aspect, the amnion, chorion, and/or intermediate layer can be laid on a suitable drying fixture prior to freeze-drying. For example, one or more strips of amnion can be laid on a suitable drying fixture. Next, chorion is laid on top of the amnion. In this aspect, an amnion/chorion tissue graft is produced. Alternatively, a strip of amnion can be placed on a first drying fixture, and a strip of chorion can be placed on a second drying fixture. The drying fixture is preferably sized to be large enough to receive the placental tissue, fully, in laid out, flat fashion. In one aspect, the drying fixture is made of Teflon® or of Delrin®, which is the brand name for an acetal resin engineering plastic invented and sold by DuPont and which is also available commercially from Werner Machine, Inc. in Marietta, Georgia. Any other suitable material that is heat- and cut- resistant, and capable of being formed into an appropriate shape to receive wet tissue can be used for the drying fixture.

Once the tissue is placed on the drying fixture, the drying fixture is placed in the freeze-dryer. The use of the freeze-dryer to dehydrate the tissue can be more efficient and thorough compared to other techniques such as thermal dehydration. In general, it is desirable to avoid ice crystal formation in the placental tissue as this may damage the extracellular matrix in the tissue. By chemically dehydrating the placental tissue prior to freeze-drying, this problem can be avoided.

In another aspect, the dehydration step involves applying heat to the tissue. In one aspect, the amnion, chorion, and/or intermediate layer is laid on a suitable drying fixture (either as individual strips or as a laminate discussed above), and the drying fixture is placed in a sterile Tyvex (or similar, breathable, heat-resistant, and sealable material) dehydration bag and sealed. The breathable dehydration bag prevents the tissue from drying too quickly. If multiple drying fixtures are being processed simultaneously, each drying fixture is either placed in its own Tyvex bag or, alternatively, placed into a suitable mounting frame that is designed to hold multiple drying frames thereon and the entire frame is then placed into a larger, single sterile Tyvex dehydration bag and sealed.

The Tyvex dehydration bag containing the one or more drying fixtures is then placed into a non-vacuum oven or incubator that has been preheated to approximately 35 to 50 degrees Celcius. The Tyvex bag remains in the oven for between 30 to 120 minutes. In one aspect, the heating step can be performed at 45 minutes at a temperature of approximately 45 °C to dry the tissue sufficiently but without over-drying or burning the tissue. The specific temperature and time for any specific oven will need to be calibrated and adjusted based on other factors including altitude, size of the oven, accuracy of the oven temperature, material used for the drying fixture, number of drying fixtures being dried simultaneously, whether a single or multiple frames of drying fixtures are dried simultaneously, and the like.

In one aspect, the placental tissue grafts described herein can be dehydrated using an innovative dehydration device which enhances the rate and uniformity of the dehydration process. In one embodiment, the drying time can be accelerated by up to 40% in one configuration of the dehydration device in comparison to conventional drying ovens. In certain aspects, the placental tissue graft is placed onto a drying fixture described herein and the drying fixture with tissue graft is inserted into the dehydration device for performing the dehydration process. In other aspects, multiple placental tissue grafts can be placed onto the drying fixture to dry more than one placental tissue grafts in the dehydration device at the same time. Although the dehydration device is useful in dehydrating the tissue grafts described herein, they can be used for dehydrating objects other than placental tissue.

Certain dehydration devices are described in U.S. Patent Pub. No. 2014/0051059, filed January 17, 2013, titled DEHYDRATION DEVICE AND METHODS FOR DRYING BIOLOGICAL MATERIALS.

### Preparation of Micronized Compositions and Pharmaceutical Compositions (Step 150)

Once the placental tissue or components thereof as described above have been dehydrated individually or in the form of a tissue graft, the dehydrated tissue(s) is micronized. The micronized compositions can be produced using instruments known in the art. For example, the Retsch Oscillating Mill MM400 can be used to produce the micronized compositions described herein. The particle size of the materials in the micronized composition can vary as well depending upon the application of the micronized composition. In one aspect, the micronized composition has particles that are less than 500 µm, less than 400 µm, less than 300 µm, less than 200 µm, less than 100 µm, less than 50 µm, less than 25 µm, less than 20 µm, less than 15 µm, less than 10 µm, less than 9 µm, less than 8 µm, less than 7 µm, less than 6 µm, less than 5 µm, less than 4 µm, less than 3 µm, less than 2 µm, or from 2 µm to 400 µm, from 25 µm to 300 µm, from 25 µm to 200 µm, or from 25 µm to 150 µm. In one aspect, the micronized composition has particles that have a diameter less than 150 µm, less than 100 µm, or less than 50 µm. In other aspects, particles having a larger diameter (*e.g.* 150 µm to 350 µm) are desirable. In all cases, the diameter of the particle is measured along its longest axis.

In one embodiment, the size of the particles may be reduced to nano-range. As one skilled in the art would understand, nanoparticles of placental components may be desirable for the increased density and/or increased release rate upon applying to the wound. Preferably, the particle size of the micronized particles is from about 0.05 µm to about 2 µm, from about 0.1 µm to about 1.0 µm, from about 0.2 µm to about 0.8 µm, from about 0.3 µm to about 0.7 µm, or from about 0.4 µm to about 0.6 µm. Alternatively, the particle size of the micronized particles is at least 0.05 µm, at least 0.1 µm, at least 0.2 µm, at least 0.3 µm, at least 0.4 µm, at least 0.5 µm, at least 0.6 µm, at least 0.7 µm, at least 0.8 µm, at least 0.9 µm, or at least 1 µm. Alternatively, the particle size of the micronized particles is less than 1 µm, less than 0.9 µm, less than 0.8 µm, less than 0.7 µm, less than 0.6 µm, less than 0.5 µm, less than 0.4 µm, less than 0.3 µm, less than 0.2 µm, less than 0.1 µm, or less than 0.05 µm.

In other aspects, particles having a range of sizes and volumes are preferred as such particles will impart differential release rates into the wound. In one embodiment, particles having a range of mass to volume ratios can be prepared by micronizing a mixture of a monolayer graft with multi-layer grafts (e.g., 2-10 layers) such that a range of graft sizes and volumes are provided. In another embodiment, particles of varying surface area to volume ratios of the same tissue material can be prepared by compressing the linear grafts into three-dimensional shapes of varying sizes (round, elliptical, oblong, etc.). As surface area to volume ratio is increased, particle dissipation increases due to the larger exposure area for endogenous enzymes, etc. This results in a faster rate of release of collagen types IV, V, and VII, cell-adhesion bio-active factors including fibronectin and laminins, and other components of the micronized particles. On the other hand, as the surface area to volume ratio is decreased, particle dissipation decreases due to the smaller exposure area for endogenous enzymes, etc. This results in a slower rate of release of collagen types IV, V, and VII, cell-adhesion bio-active factors including fibronectin and laminins and other components of the micronized particles. In combination, the use of a layer of micronized particles having different surface area to volume ratios provides for a "time-release" mechanism whereby the benefits of the micronized graft are both immediate and prolonged.

In one embodiment, the surface area to volume ratio (based on a sphere having a range of diameters as described above) is between the range of about 0.06 µm to about 6 x 10⁴ µm, about 0.06 µm to about 6 x 10³ µm, about 0.06 µm to about 6 x 10² µm, or about 0.6 µm to about 6 x 10² µm.

In one aspect, the initial micronization is performed by mechanical grinding or shredding. In another aspect, micronization is performed by cryogenic grinding. In this aspect, the grinding jar containing the tissue is continually cooled with liquid nitrogen from the integrated cooling system before and during the grinding process. Thus, the sample is embrittled and volatile components are preserved. Moreover, the denaturing of proteins in the amnion, intermediate tissue layer, and/or chorion is minimized or prevented. In one aspect, the CryoMill manufactured by Retsch can be used in this aspect.

The selection of components used to make the micronized components described herein can vary depending upon the end-use of the composition. For example, amnion and optionally chorion, intermediate tissue layer or any combination thereof can be admixed with one another and subsequently micronized. In another aspect, one or more tissue grafts composed of amnion and optionally chorion, intermediate tissue layers, or any combination thereof (*i.e*., laminates) can be micronized. In a further aspect, one or more tissue grafts composed of one or more amnion and optionally chorion, intermediate tissue layers, or any combination can be admixed with amnion and optionally chorion, intermediate tissue layer, or any combination thereof and subsequently micronized.

The amount of different components used to make the micronized compositions described herein can vary depending upon the application of the micronized composition. In one aspect, when the micronized composition is composed of amnion (with or without the intermediate tissue layer) and intermediate tissue layer, the weight ratio of amnion to intermediate tissue layer is from 10:1 to 1:10, 9:1 to 1:1, 8:1 to 1:1, 7:1 to 1:1, 6:1 to 1:1, 5:1 to 1:1, 4:1 to 1:1, 3:1 to 1:1, 2:1 to 1:1, or about 1:1. In another aspect, when the micronized composition is composed of amnion (with or without the intermediate tissue layer) and chorion, the weight ratio of chorion to amnion is from 10:1 to 1:10, 9:1 to 1:1, 8:1 to 1:1, 7:1 to 1:1, 6:1 to 1:1, 5:1 to 1:1, 4:1 to 1:1, 3:1 to 1:1, 2:1 to 1:1, or about 1:1. Regardless of the components used, the particle sizes of the micronized components is for the composition to be readily passable through the outlet, sprayer or nozzle of the device as described herein, and can be determined by a person skilled in the art. In one aspect, an anti-aggregant, such as arginine or a salt thereof, can be added to prevent aggregation of the micronized material.

Separation of particle sizes can be achieved by fractionation of the micronized material in sterile water by forming a suspension of particles. The upper most portion of the suspension will contain predominantly the smallest particles and the lower most portion of the suspension will contain predominantly the heaviest particles. Fractionation leads to particle size separation and repeated fractionation will lead to separation of the micronized particles into varying sizes. The so separated particles can be recombined in the desired ratio of particle size as is most appropriate for the wound to be treated.

In addition to the amnion, the intermediate tissue layer, and chorion, additional components can be added to the composition prior to and/or after micronization.

In another aspect, a bioactive agent can be added to the composition prior to and/or after micronization. Examples of bioactive agents include, but are not limited to, naturally occurring growth factors sourced from platelet concentrates, either using autologous blood collection and separation products, or platelet concentrates sourced from expired banked blood; bone marrow aspirate; stem cells derived from concentrated human placental cord blood stem cells, concentrated amniotic fluid stem cells or stem cells grown in a bioreactor; anti-inflammatory agents; or antibiotics. Upon application of the micronized composition with bioactive agent to the region of interest, the bioactive agent is delivered to the region over time. Thus, the micronized particles described herein are useful as delivery devices of bioactive agents and other pharmaceutical agents when administered to a subject. Release profiles can be modified based on, among other things, the selection of the components used to make the micronized compositions as well as the size of the particles.

In yet another aspect, the micronized placental components can be suspended in saline, sterile water, or any suitable buffer known in the art to form a suspension. Subsequently, extracts of the micronized placental components are prepared by separating the micronized placental particles from the solution, e.g., by way of fractionation. The obtained extract comprising growth factors and cytokines is used for direct application or injection, or is combined with other pharmaceutical products or cosmetic products. Yet, the remaining micronized placental components comprise significant amounts of growth factors for treating wound or other pharmaceutical uses.

In a further aspect, the amnion can be cross-linked with the intermediate tissue layer, chorion, or a second amnion tissue. For example, a cross-linking agent can be added to the composition (*e.g*., amnion, chorion, intermediate tissue layer, or any combination thereof as individual components and/or as tissue grafts) prior to and/or after micronization. In general, the cross-linking agent is nontoxic and non-immunogenic. When multiple components, e.g., amnion, intermediate tissue layer, and/or chorion (or a tissue graft thereof), are treated with the cross-linking agent, the cross-linking agent can be the same or different. In one aspect, the amnion, intermediate tissue layer, and chorion can be treated separately with a cross-linking agent or, in the alternative, the amnion, intermediate tissue layer, and chorion can be treated together with the same cross-linking agent. In certain aspects, the amnion, intermediate tissue layer, and chorion can be treated with two or more different cross-linking agents. The conditions for treating the amnion, intermediate tissue layer, and chorion can vary. In other aspects, the amnion, intermediate tissue layer, and/or chorion can be micronized, and the micronized composition can subsequently be treated with a cross-linking agent. In one aspect, the concentration of the cross-linking agent is from 0.1 M to 5 M, 0.1 M to 4 M, 0.1 M to 3 M, 0.1 M to 2 M, or 0.1 M to 1 M.

The cross-linking agent generally possesses two or more functional groups capable of reacting with proteins to produce covalent bonds. In one aspect, the cross-linking agent possesses groups that can react with amino groups present on the protein. Examples of such functional groups include, but are not limited to, hydroxyl groups, substituted or unsubstituted amino groups, carboxyl groups, and aldehyde groups. In one aspect, the cross-linker can be a dialdehyde such as, for example, glutaraldehyde. In another aspect, the cross-linker can be a carbodiimide such as, for example, (N-(3-dimethylaminopropyl)-N'-ethyl-carbodiimide (EDC). In other aspects, the cross-linker can be an oxidized dextran, p-azidobenzoyl hydrazide, N-[alpha-maleimidoacetoxy]succinimide ester, p-azidophenyl glyoxal monohydrate, bis-[beta-(4-azidosalicylamido)ethyl]disulfide, bis-[sulfosuccinimidyl]suberate, dithiobis[succinimidyl]propionate, disuccinimidyl suberate, and 1-ethyl-3-[3-dimethylaminopropyl]carbodiimide hydrochloride, a bifunctional oxirane (OXR), or ethylene glycol diglycidyl ether (EGDE).

In one aspect, sugar is the cross-linking agent, where the sugar can react with proteins present in the amnion, intermediate tissue layer, and chorion to form a covalent bond. For example, the sugar can react with proteins by the Maillard reaction, which is initiated by the nonenzymatic glycosylation of amino groups on proteins by reducing sugars and leads to the subsequent formation of covalent bonds. Examples of sugars useful as a cross-linking agent include, but are not limited to, D-ribose, glycerose, altrose, talose, ertheose, glucose, lyxose, mannose, xylose, gulose, arabinose, idose, allose, galactose, maltose, lactose, sucrose, cellibiose, gentibiose, melibiose, turanose, trehalose, isomaltose, or any combination thereof.

The compositions disclosed herein can be prepared using techniques known in the art. In one aspect, the composition is prepared by admixing a micronized composition described herein with a pharmaceutically-acceptable compound and/or excipient. The term "admixing" is defined as mixing the two components together so that there is no chemical reaction or physical interaction. The term "admixing" also includes the chemical reaction or physical interaction between the compound and the pharmaceutically-acceptable compound.

Surfactants (or surface-active substances) that may be present are anionic, non-ionic, cationic and/or amphoteric surfactants. Typical examples of anionic surfactants include, but are not limited to, soaps, alkylbenzenesulfonates, alkanesulfonates, olefin sulfonates, alkyl ether sulfonates, glycerol ether sulfonates, alpha-methyl ester sulfonates, sulfo fatty acids, alkyl sulphates, fatty alcohol ether sulphates, glycerol ether sulphates, fatty acid ether sulphates, hydroxy mixed ether sulphates, monoglyceride (ether) sulphates, fatty acid amide (ether) sulphates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids, e.g. acyl lactylates, acyl tartrates, acyl glutamates and acyl aspartates, alkyl oligoglucoside sulphates, protein fatty acid condensates (in particular wheat-based vegetable products) and alkyl (ether) phosphates. Examples of non-ionic surfactants include, but are not limited to, fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers or mixed formals, optionally partially oxidized alk(en)yl oligoglycosides or glucoronic acid derivatives, fatty acid N-alkylglucamides, protein hydrolysates (in particular wheat-based vegetable products), polyol fatty acid esters, sugar esters, sorbitan esters, polysorbates and amine oxides. Examples of amphoteric or zwitterionic surfactants include, but are not limited to, alkylbetaines, alkylamidobetaines, aminopropionates, aminoglycinates, imidazolinium-betaines and sulfobetaines.

In one aspect, the surfactant can be fatty alcohol polyglycol ether sulphates, monoglyceride sulphates, mono- and/or dialkyl sulfosuccinates, fatty acid isethionates, fatty acid sarcosinates, fatty acid taurides, fatty acid glutamates, alpha-olefinsulfonates, ether carboxylic acids, alkyl oligoglucosides, fatty acid glucamides, alkylamidobetaines, amphoacetals and/or protein fatty acid condensates.

Examples of zwitterionic surfactants include betaines, such as N-alkyl-N,N-dimethylammonium glycinates, for example cocoalkyldimethylammonium glycinate, N-acylaminopropyl-N,N-dimethylammonium glycinates, for example cocoacylaminopropyldimethylammonium glycinate, and 2-alkyl-3-carboxymethyl-3-hydroxyethylimidazolines having in each case 8 to 18 carbon atoms in the alkyl or acyl group, and cocoacylaminoethylhydroxyethyl-carboxymethyl glycinate.

In one aspect, the emulsifier can be a nonionogenic surfactant selected from the following: addition products of from 2 to 30 mol of ethylene oxide and/or 0 to 5 mol of propylene oxide onto linear fatty alcohols having 8 to 22 carbon atoms, onto fatty acids having 12 to 22 carbon atoms, onto alkylphenols having 8 to 15 carbon atoms in the alkyl group, and onto alkylamines having 8 to 22 carbon atoms in the alkyl radical; alkyl and/or alkenyl oligoglycosides having 8 to 22 carbon atoms in the alk(en)yl radical and the ethoxylated analogs thereof; addition products of from 1 to 15 mol of ethylene oxide onto castor oil and/or hydrogenated castor oil; addition products of from 15 to 60 mol of ethylene oxide onto castor oil and/or hydrogenated castor oil; partial esters of glycerol and/or sorbitan with unsaturated, linear or saturated, branched fatty acids having 12 to 22 carbon atoms and/or hydroxycarboxylic acids having 3 to 18 carbon atoms, and the adducts thereof with 1 to 30 mol of ethylene oxide; partial esters of polyglycerol (average degree of selfcondensation 2 to 8), trimethylolpropane, pentaerythritol, sugar alcohols (e.g. sorbitol), alkyl glucosides (e.g. methyl glucoside, butyl glucoside, lauryl glucoside), and polyglucosides (e.g. cellulose) with saturated and/or unsaturated, linear or branched fatty acids having 12 to 22 carbon atoms and/or hydroxycarboxylic acids having 3 to 18 carbon atoms, and the adducts thereof with 1 to 30 mol of ethylene oxide; mixed esters of pentaerythritol, fatty acids, citric acid and fatty alcohols and/or mixed esters of fatty acids having 6 to 22 carbon atoms, methylglucose and polyols, preferably glycerol or polyglycerol, mono-, di- and trialkyl phosphates, and mono-, di- and/or tri-PEG alkyl phosphates and salts thereof; wool wax alcohols; polysiloxane-polyalkyl-polyether copolymers and corresponding derivatives; and block copolymers, e.g. polyethylene glycol-30 dipolyhydroxystearates. In one aspect, the emulsifier is a polyalkylene glycol such as, for example, polyethylene glycol or polypropylene glycol. In another aspect, the emulsifier is polyethylene glycol having a molecular weight 100 Da to 5,000 Da, 200 Da to 2,500 Da, 300 Da to 1,000 Da, 400 Da to 750 Da, 550 Da to 650 Da, or about 600 Da.

In another aspect, the emulsifier is composed of one or more fatty alcohols. In one aspect, the fatty alcohol is a liner or branched C₆ to C₃₅ fatty alcohol. Examples of fatty alcohols include, but are not limited to, capryl alcohol (1-octanol), 2-ethyl hexanol, pelargonic alcohol (1-nonanol), capric alcohol (1-decanol, decyl alcohol), undecyl alcohol (1-undecanol, undecanol, hendecanol), lauryl alcohol (dodecanol, 1-dodecanol), tridecyl alcohol (1-tridecanol, tridecanol, isotridecanol), myristyl alcohol (1-tetradecanol), pentadecyl alcohol (1-pentadecanol, pentadecanol), cetyl alcohol (1-hexadecanol), palmitoleyl alcohol (cis-9-hexadecen-1-ol), heptadecyl alcohol (1-n-heptadecanol, heptadecanol), stearyl alcohol (1-octadecanol), isostearyl alcohol (16-methylheptadecan-1-ol), elaidyl alcohol (9E-octadecen-1-ol), oleyl alcohol (cis-9-octadecen-1-ol), linoleyl alcohol (9Z, 12Z-octadecadien-1-ol), elaidolinoleyl alcohol (9E, 12E-octadecadien-1-ol), linolenyl alcohol (9Z, 12Z, 15Z-octadecatrien-1-ol) elaidolinolenyl alcohol (9E, 12E, 15-E-octadecatrien-1-ol), ricinoleyl alcohol (12-hydroxy-9-octadecen-1-ol), nonadecyl alcohol (1-nonadecanol), arachidyl alcohol (1-eicosanol), heneicosyl alcohol (1-heneicosanol), behenyl alcohol (1-docosanol), erucyl alcohol (cis-13-docosen-1-ol), lignoceryl alcohol (1-tetracosanol), ceryl alcohol (1-hexacosanol), montanyl alcohol, cluytyl alcohol (1-octacosanol), myricyl alcohol, melissyl alcohol (1-triacontanol), geddyl alcohol (1-tetratriacontanol), or cetearyl alcohol.

The extracts obtained from the micronized compositions described herein can be lyophilized (e.g., freeze-dried) to promote stability, preserve activity and increase shelf-life. One skilled in the art would understand how to reconstitute the lyophilized product before use.

### III. Methods of Use

The compositions, kits, systems and devices described herein are useful in treating wounds. In one aspect, the wound is a result of an injury. In one aspect, the wound is a result of a surgical procedure. In one aspect, the wound has an opening on the skin. In another aspect, the wound is an internal wound on an internal organ or tissue of the subject. The composition comprising micronized placental components can be applied to the surface or inside the wound to treat the wound, such as facilitating wound healing, reducing or preventing inflammation and/or infection of the wound.

In one aspect of the disclosure, the spray composition comprising micronized compositions can be used as dermal fillers. The skin is made up of three layers: the epidermis, dermis, and subcutaneous. The epidermis is the outer layer and functions as a barrier to the external environment. The dermis is the second layer of the skin containing the structural elements, which are collagen and elastin fibers. The collagen gives the skin its strength while the elastin fibers give the skin its elasticity. In between the epidermis and dermis is an area termed the dermal-epidermal junction. It interlocks, forming fingerlike projections, called rete ridges, increasing the surface area of the epidermis that is exposed to the blood vessels in the dermis. The cells of the epidermis receive nutrients from the blood vessels in the dermis. The last layer of skin is the subcutaneous tissue which contains fat cells. These fat cells make the skin look plump and youthful. It also provides insulation to the body.

Amnion contains growth factors such as EGF, bFGF, and PDGF that promote wound healing and re-epithelialization. Not wishing to be bound by theory, the application of a topical composition composed of the micronized compositions described herein where the epithelial layer of the skin is disrupted can be effective in delivering the growth factors directly to the injured site to promote healing.

Amnion is a unique ECM due to the presence of collagen types IV, V and VII, which enables the amnion to bind water and swell. Similarly, the intermediate tissue layer of the amniotic membrane is composed largely of glycoproteins and proteoglycans, which also enables the intermediate tissue layer to bind water. Thus, the micronized particles when applied to the skin or wound help retain water in the skin, which facilitates wound healing. For example, cell migration within the wound healing cascade is facilitated in a hydrophilic environment. The intermediate layer is also composed of collagen types I, III, and IV. Type I collagen provides mechanical strength to skin by providing a major biomechanical scaffold for cell attachment and anchorage of macromolecules. Type III collagen provides elasticity. Hence, by adding the intermediate tissue layer tissue to the deep dermis it will not only increase the elasticity and scaffolding of the skin, it may make it feel softer. Another important component in the intermediate tissue layer that is beneficial to skin is proteoglycans. Proteoglycans allow the intermediate tissue layer to bind water to such a large degree and swell considerably.

In one aspect, the micronized compositions described herein are useful in enhancing or improving wound healing. The types of wounds that present themselves to physicians on a daily bases are diverse. Acute wounds are caused by surgical intervention, trauma and burns. Chronic wounds are wounds that are delayed in closing compared to healing in an otherwise healthy individual. Examples of chronic wound types plaguing patients include diabetic foot ulcers, venous leg ulcers, pressure ulcers, arterial ulcers, and surgical wounds that become infected.

The physician's goal when treating traumatic wounds is to heal the wound while allowing the patient to retain natural function in the area of the wound with minimal scaring and infection. If a wound becomes infected, it can lead to a loss of limb or life. For the most part, physicians heal these patients without incident. However, physicians dealing with chronic wounds are mainly concerned with closing the wound as quickly as possible to minimize the risk of an infection that could lead to loss of limb or life. Chronic wounds are wounds on patients that have comorbidities that complicate or delay the healing cascade. In one aspect, the micronized compositions described herein can function as a tissue regeneration template that delivers essential wound healing factors, extracellular matrix proteins and inflammatory mediators to help reduce inflammation, enhance healing, and reduce scar tissue formation. In this aspect, the micronized placental compositions described herein are used in treating wounds amenable to negative pressure technology, including burns and ulcers, e.g., chronic ulcers, diabetic ulcers, decubitus ulcers and the like.

In another aspect, the micronized placental tissue is used in conjunction with conventional treatments, including, but not limited to, negative pressure therapy, and may further be used in combination with matrices or scaffolds comprised of biocompatible materials, such as collagen, hyaluronic acid, gelatin or combinations thereof.

In another aspect, the micronized compositions described herein can be used to enhance wound healing and prevent scar formation as a result of a surgical incision. In one aspect, the micronized composition can be applied to the open incision followed by suturing the incision. For example, the micronized composition can be applied directly to the open incision by sprinkling the composition within the incision or by injecting the composition into the incision using a syringe, a small bellows device, or other related device. The micronized compositions are particularly useful where large incisions are produced by a surgical procedure. An example of such a procedure involves the treatment of spinal scoliosis, which requires a significant incision along the back of the subject. In one aspect, micronized compositions composed of an amnion/chorion laminate where the epithelium layer is intact are useful in the healing of surgical incisions with minimal scarring. With respect to wound healing and the prevention of scar formation, the micronized compositions described herein can be used in combination with other wound healing products. For example, any of the tissue grafts described herein that are a precursor to the micronized compositions can be applied to the wound after the micronized composition and adhesive or gelation agent have been applied.

The compositions described herein are useful in preventing or reducing scar formation on the spine or near the spine and sealing dural tears.

Depending upon the surgical procedure, the micronized composition can be applied directly to the spinal dura, the surrounding region of the spine to include nerve roots, or a combination thereof. Due to the unique structure of vertebrae, the micronized composition can be placed and affixed at the appropriate position in the subject. The micronized compositions can also provide proximal and distal barrier coverage where the spinal lamina has been removed for exposure to the affected area.

The micronized compositions are useful in preventing or reducing scar formation that can result from a variety of surgical procedures associated with the spine. The micronized compositions can be used after any procedure in the neck, mid-back, or lower back. Depending upon the application, the epithelium of the amnion can be substantially removed. For example, in posterior procedures such as a laminectomy or discectomy, the epithelium layer of the amnion is substantially removed. Removal of the epithelial cell layer exposes the amnion's basement membrane layer, which increases cell signaling characteristics. This up regulation response enhances cellular migration and expression of anti-inflammatory proteins, which inhibits fibrosis. The spinal dura is typically left unprotected following posterior procedures.

In other aspects of the disclosure, the epithelial cell layer of the amnion is not removed. For example, in anterior procedures or modified anterior procedures such as Anterior Lumbar Interbody Fusion (ALIF) and Transforaminal Interbody Fusion (TLIF), the amnion epithelium layer is not removed and remains intact. In these aspects of the disclosure, the micronized compositions provide additional protection to the vertebral surgical site by maintaining separation from the peritoneum, larger vessels, and abdominal musculature. The micronized composition serves as a reduced friction anatomical barrier against adhesions and scaring. For example, the micronized composition can prevent scar tissue from binding major blood vessels to the spine. This is a common problem with post-spinal surgery, and a second surgical procedure is required to address the problem.

The micronized composition in combination with a gelation agent described herein can be used in general surgery procedures. For example, general surgical procedures include procedures related to the abdominal cavity. These include the intestines, stomach, colon, liver, gallbladder, appendix, bile ducts and thyroid glands. Procedures may include hernias, polypectomy, cancer removal, surgical treatment of Crohn's and ulcerative colitis. These procedures may be done open or laparoscopically. In other aspects, the micronized compositions can be used to facilitate closure of anastomosis, an anti-adhesion barrier for anastomosis, or an anti-adhesion barrier for hernia repair.

### IV. Apparatuses and Methods for Application

As described above, the micronized composition is injected onto or into a location of treatment. In some such embodiments of the disclosure, in order to provide an additional layer of protection and to seal the micronized composition onto the location of treatment, the gelation agent is applied over or in combination with the micronized composition.

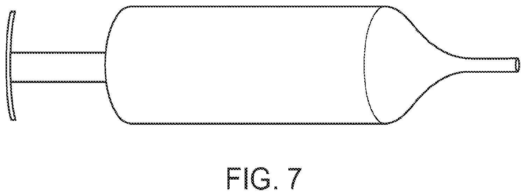

The micronized composition and the adhesive or gelation agent composition are in flowable form. In one embodiment, each composition is a liquid, a gel, a thixotropic agent, or a paste. Each may be stored separately and applied sequentially. For example, each of the micronized composition and the gelation agent composition may be stored in separate bottles or other containers, such as, for example, the container shown in Figure 7. The container may include a syringe, which is depressible, such as shown in Figure 7. In such embodiments, pressure is created inside the container when the syringe is depressed, which causes the flowable composition inside the container to be extruded from the proximal tip of the container nozzle.

In other embodiments, as described above in the previous section, a combination formulation may be created, which comprises a flowable (e.g., liquid or gel-like) formulation formed of both the micronized placental tissue components and an adhesive or gelation agent. In embodiments containing both the micronized placental tissue and sealant in a single combination, any suitable application apparatus, such as the apparatus described above and depicted in Figure 7 may be used to apply the formulation to a subject.

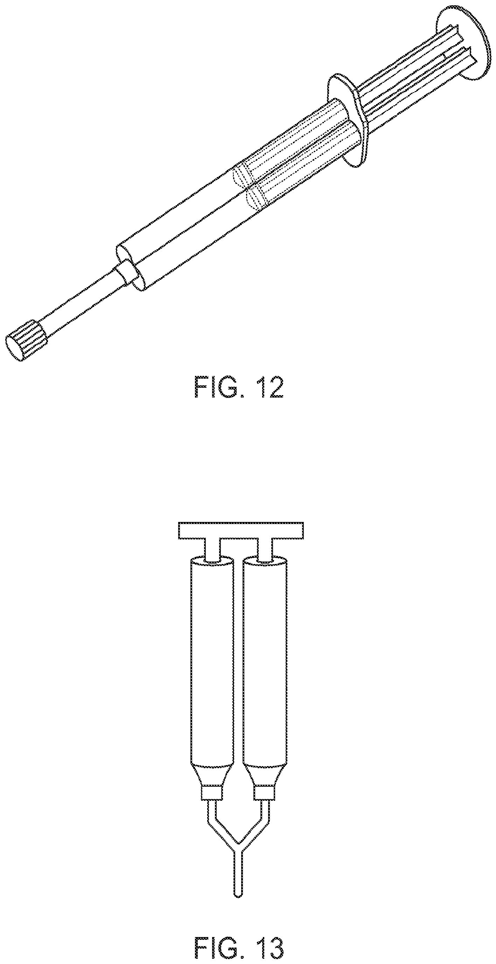

As described above, in preferred embodiments, simultaneous application of the micronized placental tissue composition and the adhesive or gelation agent composition is desired; however, some embodiments of the two compositions are reactive and must be stored separately. In such embodiments, a dual delivery device may be used. Non-limiting examples of dual delivery devices are depicted in Figures 12-13.

Figures 12-13 depict examples of a double syringe, which may be used to simultaneously administer a micronized placental tissue composition and an adhesive or gelation agent composition to a subject. A first chamber or vial of the syringe is provided with the micronized placental tissue composition and a second chamber is provided with the adhesive or gelation agent composition (or vice versa). The first chamber is connected to a first delivery tube or needle and a second chamber is connected to a second delivery tube or needle. An aperture is disposed at a proximal tip of each delivery tube or needle.

In at least some embodiments, the two chambers are disposed side by side, and the two delivery tubes or needles are disposed side by side. Each chamber is provided with a plunger. When depressed, each plunger moves proximally, decreasing the volume of each chamber, and thereby causing the contents of each chamber to be expelled through the delivery tube or needle and out the proximal aperture. In such embodiments, the two plungers are connected, for example, via a shared top surface, to facilitate simultaneous and equal movement of the plungers. Such a feature may help ensure equal application of the micronized placental tissue composition and the adhesive or gelation agent composition. In some embodiments, an equal ratio may not be desired. In such embodiments, the size of the chambers and/or the diameter of the delivery tubes or needles may vary so that a desired ratio of micronized placental tissue composition to adhesive or gelation agent composition is expelled from the syringe upon depression of the plungers.

Optionally, in some embodiments of a double syringe, an applicator is connected to a proximal end of the delivery tubes or needles to facilitate application and/or mixing of the flowable compositions. For example, in some embodiments, a brush or scraper is disposed at the proximal end (see Figure 12). In such embodiments, depressing the syringe causes the micronized placental tissue composition and the adhesive or gelation agent composition to be expelled onto the applicator. The applicator can then be brushed or gentle scraped against the subject to apply and combine the two compositions. In other embodiments, the applicator is a connector, which is attached at its distal end to the proximal ends of both the first and second delivery tubes or needles, and which defines a single lumen at its proximal end (see Figure 13). In such embodiments, mixing of the compositions may occur within the connector before being expelled from the proximal end of the single lumen.

## Claims

1. A composition formulated for injection comprising micronized amnion, optionally further comprising micronized placental tissue selected from the group consisting of chorion, intermediate tissue layer, or any combination thereof and a biocompatible gelation agent in a suitable excipient, wherein the biocompatible gelation agent comprises:
a thermosensitive sol-gel reversible hydrogel, preferably
a poloxamer, wherein the composition is in a sol (liquid) phase at about 0 °C to about 20 °C and transitions to a gel (solid) phase at about 25 °C to about 40 °C.

2. The composition of claim 1, wherein the composition is a liquid, a gel, or a paste prior to application to a subject.

3. The composition of claim 1 or 2, wherein the composition becomes a solid, a semi-solid, or a gel after application to a subject.

4. The composition of any one of claims 1-3, wherein the micronized placental tissue and the biocompatible gelation agent are mixed immediately prior to injection.

5. The composition of any one of claims 1-4, wherein the micronized amnion and optional micronized placental tissue have a particle size of less than 100µm.

6. A system for injecting micronized amnion, the system comprising:
a first composition comprising micronized amnion, optionally further comprising placental tissue selected from the group consisting of micronized chorion, intermediate tissue layer, or any combination thereof, wherein the first composition is in a flowable form;
a second composition comprising a biocompatible gelation agent in a suitable excipient, wherein the second composition is in a flowable form; and
an administration device, comprising a first chamber having the first composition disposed therein, a second chamber having the second composition stored therein, and one or more apertures configured to expel the first and second compositions therefrom;
wherein the biocompatible gelation agent comprises:
a thermosensitive sol-gel reversible hydrogel, preferably
a poloxamer, wherein the composition is in a sol (liquid) phase at about 0 °C to about 20 °C and transitions to a gel (solid) phase at about 25 °C to about 40 °C.

7. The system of claim 6, wherein the composition is a liquid, a gel, or a paste prior to application to a subject.

8. The system of claim 6 or 7, wherein the micronized placental tissue has a particle size of less than 100µm.

9. The system of any one of claims 6-8, wherein the administration device comprises a double syringe.

10. The system of claim 6, configured so that the first and second compositions mix within a chamber or tube prior to expulsion from a proximal aperture of the administration device.

11. The system of claim 6, configured so that the first composition is expelled from a first nozzle or aperture and the second composition is expelled from a second nozzle or aperture, and so that mixing of the first and second compositions occurs only external to the administration device.

## Patentansprüche

1. Zusammensetzung, die zur Injektion formuliert ist, die mikronisiertes Amnion umfasst, optional ferner umfassend mikronisiertes Plazentagewebe, das aus der Gruppe ausgewählt ist, die aus Chorion, Zwischengewebeschicht oder einer beliebigen Kombination davon besteht, und ein biokompatibles Geliermittel in einem geeigneten Hilfsstoff, wobei das biokompatible Geliermittel Folgendes umfasst:
ein wärmeempfindliches reversibles Sol-Gel-Hydrogel, bevorzugt
ein Poloxamer, wobei die Zusammensetzung bei etwa 0 °C bis etwa 20 °C in einer Solphase (flüssig) ist und bei etwa 25 °C bis etwa 40 °C in eine Gelphase (fest) übergeht.

2. Zusammensetzung nach Anspruch 1, wobei die Zusammensetzung vor Anwendung an einem Subjekt eine Flüssigkeit, ein Gel oder eine Paste ist.

3. Zusammensetzung nach Anspruch 1 oder 2, wobei die Zusammensetzung nach Anwendung an einem Subjekt zu einem Feststoff, einem Halbfeststoff oder einem Gel wird.

4. Zusammensetzung nach einem der Ansprüche 1-3, wobei das mikronisierte Plazentagewebe und das biokompatible Geliermittel unmittelbar vor der Injektion gemischt werden.

5. Zusammensetzung nach einem der Ansprüche 1-4, wobei das mikronisierte Amnion und das optionale mikronisierte Plazentagewebe eine Partikelgröße von weniger als 100 µm aufweisen.

6. System zum Injizieren von mikronisiertem Amnion, wobei das System Folgendes umfasst:
eine erste Zusammensetzung, die mikronisiertes Amnion umfasst, optional ferner umfassend Plazentagewebe, das aus der Gruppe ausgewählt ist, die aus mikronisiertem Chorion, Zwischengewebeschicht oder einer beliebigen Kombination davon besteht, wobei die erste Zusammensetzung in einer fließbaren Form ist;
eine zweite Zusammensetzung, die ein biokompatibles Geliermittel in einem geeigneten Hilfsstoff umfasst, wobei die zweite Zusammensetzung in einer fließbaren Form ist; und
eine Verabreichungsvorrichtung, die eine erste Kammer, welche die darin angeordnete erste Zusammensetzung aufweist, eine zweite Kammer, welche die darin gespeicherte zweite Zusammensetzung aufweist, und eine oder mehrere Öffnungen umfasst, die konfiguriert sind, um die erste und die zweite Zusammensetzung daraus auszustoßen;
wobei das biokompatible Geliermittel Folgendes umfasst:
ein wärmeempfindliches reversibles Sol-Gel-Hydrogel, bevorzugt
ein Poloxamer, wobei die Zusammensetzung bei etwa 0 °C bis etwa 20 °C in einer Solphase (flüssig) ist und bei etwa 25 °C bis etwa 40 °C in eine Gelphase (fest) übergeht.

7. System nach Anspruch 6, wobei die Zusammensetzung vor Anwendung an einem Subjekt eine Flüssigkeit, ein Gel oder eine Paste ist.

8. System nach Anspruch 6 oder 7, wobei das mikronisierte Plazentagewebe eine Partikelgröße von weniger als 100 µm aufweist.

9. System nach einem der Ansprüche 6-8, wobei die Verabreichungsvorrichtung eine Doppelspritze umfasst.

10. System nach Anspruch 6, das konfiguriert ist, sodass sich die erste und die zweite Zusammensetzung vor dem Ausstoß aus einer proximalen Öffnung der Verabreichungsvorrichtung innerhalb einer Kammer oder eines Röhrchens vermischen.

11. System nach Anspruch 6, das konfiguriert ist, sodass die erste Zusammensetzung aus einer ersten Düse oder Öffnung ausgestoßen wird und die zweite Zusammensetzung aus einer zweiten Düse oder Öffnung ausgestoßen wird, und sodass das Vermischen der ersten und der zweiten Zusammensetzung nur außerhalb der Verabreichungsvorrichtung stattfindet.

## Revendications

1. Composition formulée pour injection comprenant de l'amnios micronisé, éventuellement comprenant en outre un tissu placentaire micronisé choisi dans le groupe constitué de chorion, d'une couche de tissu intermédiaire ou de toute combinaison de ceux-ci et d'un agent de gélification biocompatible dans un excipient approprié, dans lequel l'agent de gélification biocompatible comprend :
un hydrogel réversible sol-gel thermosensible, de préférence
un poloxamère, dans laquelle la composition est en phase sol (liquide) à environ 0 °C à environ 20 °C et passe en phase de gel (solide) à environ 25 °C à environ 40 °C.

2. Composition selon la revendication 1, dans laquelle la composition est un liquide, un gel ou une pâte avant application à un sujet.

3. Composition selon la revendication 1 ou 2, dans laquelle la composition devient un solide, un semi-solide ou un gel après application à un sujet.

4. Composition selon l'une quelconque des revendications 1 à 3, dans laquelle le tissu placentaire micronisé et l'agent de gélification biocompatible sont mélangés immédiatement avant l'injection.

5. Composition selon l'une quelconque des revendications 1 à 4, dans laquelle l'amnios micronisé et le tissu placentaire micronisé éventuel ont une taille de particule inférieure à 100 µm.

6. Système d'injection d'amnios micronisé, le système comprenant :
une première composition comprenant de l'amnios micronisé, comprenant éventuellement en outre un tissu placentaire choisi dans le groupe constitué de chorion micronisé, d'une couche de tissu intermédiaire ou de toute combinaison de ceux-ci, dans lequel la première composition est sous une forme fluide ;
une seconde composition comprenant un agent de gélification biocompatible dans un excipient approprié, dans lequel la seconde composition est sous une forme fluide ; et
un dispositif d'administration, comprenant une première chambre dans laquelle est disposée la première composition, une seconde chambre dans laquelle est stockée la seconde composition, et une ou plusieurs ouvertures conçues pour en expulser les première et seconde compositions ;
dans lequel l'agent de gélification biocompatible comprend :
un hydrogel réversible sol-gel thermosensible, de préférence
un poloxamère, dans lequel la composition est en phase sol (liquide) à environ 0 °C à environ 20 °C et passe en phase de gel (solide) à environ 25 °C à environ 40 °C.

7. Système selon la revendication 6, dans lequel la composition est un liquide, un gel ou une pâte avant l'application à un sujet.

8. Système selon la revendication 6 ou 7, dans lequel le tissu placentaire micronisé a une taille de particule inférieure à 100 µm.

9. Système selon l'une quelconque des revendications 6 à 8, dans lequel le dispositif d'administration comprend une double seringue.

10. Système selon la revendication 6, conçu de sorte que les première et seconde compositions se mélangent à l'intérieur d'une chambre ou d'un tube avant l'expulsion d'une ouverture proximale du dispositif d'administration.

11. Système selon la revendication 6, conçu de sorte que la première composition est expulsée d'une première buse ou ouverture et la seconde composition est expulsée d'une seconde buse ou ouverture, et de sorte que le mélange des première et seconde compositions se produit uniquement à l'extérieur du dispositif d'administration.
